# EUROPEAN PATENT SPECIFICATION

(11) **EP 2 508 171 B1**
(45) Date of publication and mention of the grant of the patent: **07.05.2014**
(21) Application number: 11159810.8
(22) Date of filing: 25.03.2011
(51) Int. Cl.: A61K 41/00, A61K 47/48, A61K 33/26, A61K 31/122, A61K 9/14, A61K 9/51, A61K 47/02, A61K 49/18, A61P 35/00

(54) **Pharmaceutical magnetic nano-particle composition**
Pharmazeutische Zusammensetzung mit magnetischen Nanopartikeln
Composition pharmaceutique à base de nanoparticules magnétiques

(43) Date of publication of application: 10.10.2012
(73) Proprietor: Universitätsklinikum Erlangen, 91054 Erlangen (DE); Friedrich-Alexander-Universität Erlangen-Nürnberg, 91054 Erlangen (DE)
(72) Inventor: Schreiber, Eveline, 90552 Röthenbach / Pegnitz (DE); Tietze, Rainer, 91058 Erlangen (DE); Alexiou, Christoph, 91052 Erlangen (DE); Lyer, Stefan, 91052 Erlangen (DE)
(74) Representative: TBK

(56) References cited:
- EP-B1- 0 971 692
- WO-A1-2010/134087
- WO-A2-02/43708
- US-A1- 2010 303 730
- ZBORIL R ET AL: "One-step solid state synthesis of capped [gamma]-Fe2O 3 nanocrystallites", NANOTECHNOLOGY 20080205 GB LNKD- DOI:10.1088/0957-4484/19/9/095602, vol. 19, no. 9, 5 February 2008 (2008-02-05), XP7919078, ISSN: 0957-4484
- JAIN T K ET AL: "Magnetic nanoparticles with dual functional properties: Drug delivery and magnetic resonance imaging", BIOMATERIALS, ELSEVIER SCIENCE PUBLISHERS BV., BARKING, GB, vol. 29, no. 29, 1 October 2008 (2008-10-01), pages 4012-4021, XP023611046, ISSN: 0142-9612, DOI: DOI:10.1016/J.BIOMATERIALS.2008.07.004 [retrieved on 2008-07-22]
- CHRISTOPH ALEXIOU ET AL: "Targeting cancer cells: magnetic nanoparticles as drug carriers", EUROPEAN BIOPHYSICS JOURNAL ; WITH BIOPHYSICS LETTERS, SPRINGER, BERLIN, DE, vol. 35, no. 5, 1 May 2006 (2006-05-01), pages 446-450, XP019331553, ISSN: 1432-1017, DOI: DOI:10.1007/S00249-006-0042-1
- LYER S ET AL: "Visualisation of tumour regression after local chemotherapy with magnetic nanoparticles - A pilot study", ANTICANCER RESEARCH 2010 INTERNATIONAL INSTITUTE OF ANTICANCER RESEARCH GRC, vol. 30, no. 5, May 2010 (2010-05), pages 1553-1557, XP9150293, ISSN: 0250-7005
- MISHRA B ET AL: "Colloidal nanocarriers: a review on formulation technology, types and applications toward targeted drug delivery", NANOMEDICINE: NANOTECHNOLOGY, BIOLOGY AND MEDICINE, ELSEVIER, NL, vol. 6, no. 1, 1 February 2010 (2010-02-01), pages 9-24, XP027501929, ISSN: 1549-9634, DOI: DOI:10.1016/J.NANO.2009.04.008 [retrieved on 2010-02-01]
- GARCIA-ALONSO A ET AL: "Assessment of some parameters involved in the gelatinization and retrogration of starch", FOOD CHEMISTRY 199908 GB, vol. 66, no. 2, August 1999 (1999-08), pages 181-187, ISSN: 0308-8146
- HICKMAN B ELLIOT ET AL: "Autoclave and beta-Amylolysis Lead to Reduced in Vitro Digestibility of Starch", JOURNAL OF AGRICULTURAL AND FOOD CHEMISTRY, vol. 57, no. 15, August 2009 (2009-08), pages 7005-7012, ISSN: 0021-8561
- Mikhail V. Avdeev ET AL: "Structure and in Vitro Biological Testing of Water-Based Ferrofluids Stabilized by Monocarboxylic Acids", Langmuir, vol. 26, no. 11, 1 June 2010 (2010-06-01), pages 8503-8509, XP055049181, ISSN: 0743-7463, DOI: 10.1021/la904471f
- GIRI ET AL: "Synthesis and characterizations of water-based ferrofluids of substituted ferrites [Fe1-xBxFe2O4, B=Mn, Co (x=0-1)] for biomedical applications", JOURNAL OF MAGNETISM AND MAGNETIC MATERIALS, ELSEVIER SCIENCE PUBLISHERS, AMSTERDAM, NL, vol. 320, no. 5, 26 December 2007 (2007-12-26), pages 724-730, XP022401908, ISSN: 0304-8853
- REGMI RAJESH ET AL: "Effects of fatty acid surfactants on the magnetic and magnetohydrodynamic properties of ferrofluids", JOURNAL OF APPLIED PHYSICS, AMERICAN INSTITUTE OF PHYSICS. NEW YORK, US, vol. 106, no. 11, 1 December 2009 (2009-12-01), pages 113902-113902, XP012127272, ISSN: 0021-8979, DOI: 10.1063/1.3259382
- PRADHAN ET AL: "Cellular interactions of lauric acid and dextran-coated magnetite nanoparticles", JOURNAL OF MAGNETISM AND MAGNETIC MATERIALS, ELSEVIER SCIENCE PUBLISHERS, AMSTERDAM, NL, vol. 311, no. 1, 15 March 2007 (2007-03-15), pages 282-287, XP022036675, ISSN: 0304-8853, DOI: 10.1016/J.JMMM.2006.10.1181
- Rainer Tietze ET AL: "Mitoxantrone Loaded Superparamagnetic Nanoparticles for Drug Targeting: A Versatile and Sensitive Method for Quantification of Drug Enrichment in Rabbit Tissues Using HPLC-UV", Journal of Biomedicine and Biotechnology, vol. 50, no. 11-12, 1 January 2010 (2010-01-01), pages 705-9, XP055049205, ISSN: 1110-7243, DOI: 10.1016/S0021-9673(02)01536-4

## Description

### Technical Field

The present invention relates to a pharmaceutical composition comprising magnetic nano-particles for magnetic targeting of drugs. The invention further relates to the use of said pharmaceutical composition as a medicament and specifically for use in cancer treatment by magnetic drug targeting using an external magnetic field. The present invention also relates to a method for manufacturing said pharmaceutical composition.

### Background art

The therapy of diseases highly depends on the selectivity of a therapeutic agent and is therefore a balancing between the efficacy and toxicity of each therapeutic agent. This specifically accounts for therapies using therapeutic agents which need to be delivered to regional and local areas of a subject and even more in case the agent has a high toxicity such as chemotherapeutics.

Therefore, in case of a regional or local disease there is a need to administer therapeutic and/or imaging agents selectively into the regional or local disease areas. Such a selective administration can thus lower the systemic dosage of a therapeutic agent in order to reduce or prevent side effects. Hence, drug carrier systems are needed which are suitable for selectively delivering a therapeutic agent to a target tissue of a subject. Also imaging agents are needed to identify a local disease and to control the success of a local therapy thereby making is possible to further decrease the dosage of a therapeutic agent. Accordingly, there is a demand to reduce side effects caused by a systemic therapy while increasing the efficacy of the therapy by taking advantage of suitable drug carrying system for regional or local therapy preferably accompanied by monitoring the onset and progress of a therapy.

Various magnetic nano-particle compositions have been described for targeted therapy and monitoring. Also different biocompatible coatings have been described for magnetic nano-particles including biopolymers such as dextrane and starch, resin coatings, such as polystyrene and polyethylenglycol (PEG), as well as inorganic coatings, such as gold, silica or alumina. Also nano-particle compositions have been described, which are used to carry a drug by exhibiting biopolymer coatings which may bind certain drugs via covalent binding or by ionic binding via ionic functional groups of the biopolymer.

However, the prior art nano-particle compositions have certain drawbacks. For instance, the known compositions may require laborious synthesis, are expensive, or are limited to a specific application field. If acting as a drug or imaging agent carrier, the prior art nano-particles may have coatings that do not satisfy high demands for instance on stability, loading capability and sterility. At present, there is no coated nano-particle composition available that can suitably be used for various application fields without laborious individualized modifications, while satisfying high demands on characteristics such as stability, loading capability and sterility of the composition.

US 2010303730 (A1) relates to a method of making iron-containing nanoparticles that includes contacting an iron-containing precursor with a reducing agent at a temperature less than 200°C and allowing the mixture to react to form magnetite nanoparticles. The nanoparticles may be surface modified by surfactants and/or capping agents, such as PEG and oleic acid and form stable dispersions in non-aqueous media.

R. Zboril et al. ("One-step solid state synthesis of capped γ-Fe2O3 nanocrystallites", Nanotechnology, vol. 19, no. 9, 05.02.2008) describes solid state synthesis of soluble maghemite upon reaction with aliphatic acid, such as palmitic acid, lauric acid, or oleylamine leading to a product being soluble in many organic solvents or dispersible in organophilic solvents.

WO 2010/134087 (A1) relates to an aqueous dispersible magnetic nanoparticle formulation with nanoparticles being composed of an iron oxide core coated with the long chain polymer lipid glyceryl monooleate, which provides aqueous dispersibility without use of surfactant.

EP 0971692 (A2) relates to specific magnetosomes consisting of a magnetic iron oxide magnetite monocrystal and a phospholipid membrane surrounding said crystal.

T.K. Jain et al. (Biomaterials, Volume 29, Issue 29, October 2008, Pages 4012-4021) describes oleic acid-coated iron-oxide and pluronic-stabilized magnetic nanoparticles for drug delivery and magnetic resonance imaging properties.

WO 02/43708 (A2) relates to magnetic particles that comprise a magnetic substance combined with a therapeutic substance. The particles are coated with phosphate group-modified starch polymers and mitoxantrone is attached to the phosphate groups via reversible ionic interaction. The particles are used for the regional magnetotherapy of diseases by intra-arterial application.

C. Alexiou et al. (European Biophysics Journal May 2006, Volume 35, Issue 5, pp 446-450) describes magnetic drug targeting employing iron oxide nanoparticles covered by starch derivatives with phosphate groups which bound mitoxantrone as chemotherapeutikum.

S. Lyer et al. ("Visualisation of tumour regression after local chemotherapy with magnetic nanoparticles - A pilot study", ANTICANCER RESEARCH, vol. 30, no. 5, pages 1553-1557) relates to the visualization of the vascularization and tumor size by DYNA-CT providing information applicable for magnetic drug targeting.

B. Mishra et al. (Nanomedicine: Nanotechnology, Biology and Medicine, Volume 6, Issue 1, pages 9-24) reviews colloidal nanocarriers with respect to formulation aspects, types, and site-specific drug targeting using various forms of colloidal nanocarriers with special insights to the field of oncology. Specialized nanotechnological approaches like quantum dots, dendrimers, integrins, monoclonal antibodies, which have been extensively researched for targeted delivery of therapeutic and diagnostic agents, are also discussed. M. V. Avdeev ("Structure and in Vitro Biological Testing of Water-Based Ferrofluids Stabilized by Monocarboxylic Acids", Langmuir, 2010, 26 (11), pp 8503-8509) describes water-based ferrofluids with double-layer steric stabilization by lauric and myristic acids.

J. Giri et al. ("Synthesis and characterizations of water-based ferrofluids of substituted ferrites [Fe1-xBxFe2O4, B=Mn, Co (x=0-1)] for biomedical applications", JOURNAL OF MAGNETISM AND MAGNETIC MATERIALS, vol. 320, no. 5, 2008, pages 724-730) describes ferrofluids having lauric acid coated magnetic nanoparticles.

R. Regmi et al. (J. Appl. Phys. 106, 113902 (2009)) describes Fe₃O₄ magnetic nanoparticles having diameters of approximately 12 nm, which were coated with three different fatty acid surfactants, oleic acid, lauric acid, and myristic acid, which were dissolved in cyclohexane for studies of the magnetohydrodynamics.

P. Pradhan et al. (Journal of Magnetism and Magnetic Materials, Vol. 311, Issue 1, 2007, pages 282-287) describes *in vitro* cytocompatibility and cellular interactions of lauric acid and dextran-coated magnetite nanoparticles, which were evaluated with two different cell lines (mouse fibroblast and human cervical carcinoma). Lauric acid-coated magnetite nanoparticles were less cytocompatible than dextran-coated magnetite nanoparticles and cellular uptake of lauric acid-coated magnetic nanoparticles was more than that of dextran-coated magnetite nanoparticles.

R. Tietze et al. (J. Biomed. Biotechnol., vol. 50, no. 11-12, 1 January 2010, pages 705-9) describes an analytical method (HPLC-UV) to detect pure or ferrofluid-bound mitoxantrone in a complex matrix even in trace amounts in order to perform biodistribution studies.

### Summary of the invention

The present invention has been made in the light of the foregoing circumstances and an object of the present invention is to provide a cheap, straight forward accessible and efficient pharmaceutical composition comprising coated nano-particles, which is used as a carrier for drugs in a straight forward manner. The pharmaceutical composition has the benefit that it can be easily and highly efficiently modified with a therapeutic agent under standardized conditions so that it is suitable for therapy by means of magnetic targeting. A further object of the present invention is to provide a method for manufacturing said pharmaceutical composition.

The objects of the present invention are attained by the following items concerning the subject-matter of the invention and including particularly efficient modifications:
1. A pharmaceutical composition for magnetic targeting, the composition comprising magnetic nano-particles, an aqueous dispersion medium having a solids content ranging from 8 to 25 mg/ml, the particles comprising a magnetic particle core and a coating comprising a fatty acid, wherein the fatty acid is lauric acid, and wherein the particle cores are coated with 25 to 45 parts by weight of the fatty acid relative to 100 parts by weight of the particle core, wherein the particle core is an iron oxide, preferably mono- and multi-domain Fe₂O₃/Fe₃O₄ and wherein the magnetic particles further comprise mitoxantrone, which is adhered directly onto the surface of the particle cores and/or into the inside of an aggregate of the particle cores by electrostatic interaction.
2. A pharmaceutical composition according to item 1, wherein the particle cores are coated with 25 to 35 parts by weight of the fatty acid relative to 100 parts by weight of the particle core.
3. A pharmaceutical composition according to any of items 1 or 2, wherein the magnetic particles have a zeta-potential in an aqueous dispersion medium of from -15 to -45 mV, preferably -20 to -35 mV.
4. A pharmaceutical composition according to any of items 1 to 3, wherein the particles are loaded with mitoxantrone with at least 0.1 parts by weight, preferably at least 1 parts by weight, particularly preferably at least 2 parts by weight, relative to 100 parts by weight of the particle core.
5. A pharmaceutical composition according to any of items 1 to 4, wherein the magnetic particles have a particle size of less than 220 nm, preferably less than 180 nm, as measured by hydro-dynamic laser scattering in a mono- or multi-dispersed state in an aqueous medium.
6. A pharmaceutical composition according to any of items 1 to 5 for use as a medicament.
7. A pharmaceutical composition according to any of items 1 to 5, for use in cancer treatment by magnetic drug targeting using an external magnetic field.
8. A pharmaceutical composition for use according to item7, wherein the composition is administered intra-arterial in a therapeutically effective amount.
9. A pharmaceutical composition for use according to item 7 or 8, wherein the positioning and the vascularization of the target tissue and the positioning for accessing the arterial system are determined by angiographic imaging, optionally by Dyna-CT.
10. A pharmaceutical composition for use according to any of item 7 to 9, wherein the amount of the pharmaceutical composition to be administered is adjusted by means of on-line monitoring the target tissue and/or wherein the composition is administered by one-time administration or by repeated administration preferably following angiographic and MRT-based monitoring.
11. A pharmaceutical composition for use according to any of items 7 to 10, wherein the pharmaceutical composition is guided and accumulated to the target tissue by application of an external magnetic field gradient, preferably having a high magnetic flux density ranging from 0.5 to 1.5 T and a magnetic field gradient ranging from 10 to 90 T/m.
12. A pharmaceutical composition for use according to any of items 7 to 11, wherein the magnetic field is a multidirectional magnetic field being spatially variable, preferably by means of different pole shoes.
13. Method for manufacturing a pharmaceutical composition according to any of items 1 to 12, the method comprising at least the steps:
   - producing magnetic iron oxide nano-particles as said particle cores,
   - coating said particle cores with lauric acid at least once, and
   - loading mitoxantrone onto the surface of the particles cores and/or into the inside of an aggregate of the particles cores by electrostatic interaction.
14. Method for manufacturing a pharmaceutical composition according to item 13, wherein the nano-particles are iron oxide particles which are precipitated in an aqueous solution containing iron-(II) and iron-(III) salts by adding a base.
15. Method for manufacturing a pharmaceutical composition according to item 14, wherein the iron-(II) and iron-(III) salts are iron chlorides or sulfates, preferably at a molar ratio of 2:3.
16. Method for manufacturing a pharmaceutical composition according to any of items 13 to 15, wherein the magnetic-nanoparticle cores are coated with lauric acid at an elevated temperature of 60°C or more, preferably 90°C or more, preferably in an aqueous suspension.
17. Method for manufacturing a pharmaceutical composition according to item 16, wherein the elevated temperature is held constant for a predetermined time followed by slow cooling.
18. Method for manufacturing a pharmaceutical composition according to any of items 13 to 17, wherein the method further comprises a step of autoclavation of the composition.
19. Method for manufacturing a pharmaceutical composition according to any of items 13 to 18, wherein the method further comprises the step of separating the nano-particles so as to eliminate particles and/or particle agglomerates having a size of 220 nm or more, preferably of 180 nm or more.

### Description of the Drawings

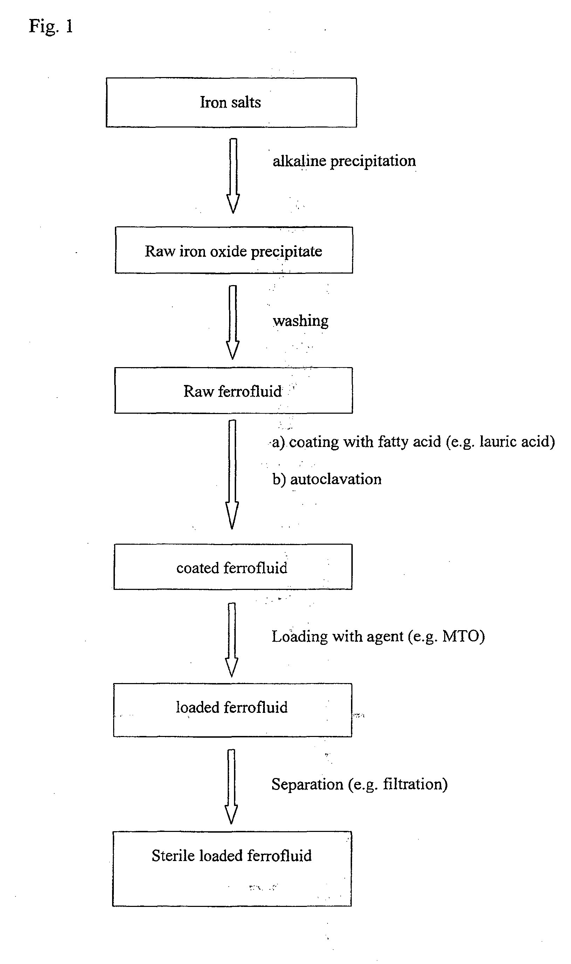

Fig. 1 is to schematic illustration of an exemplified nano-particle composition synthesis including the functionalization with an agent, e.g. mitoxantron (MTO).

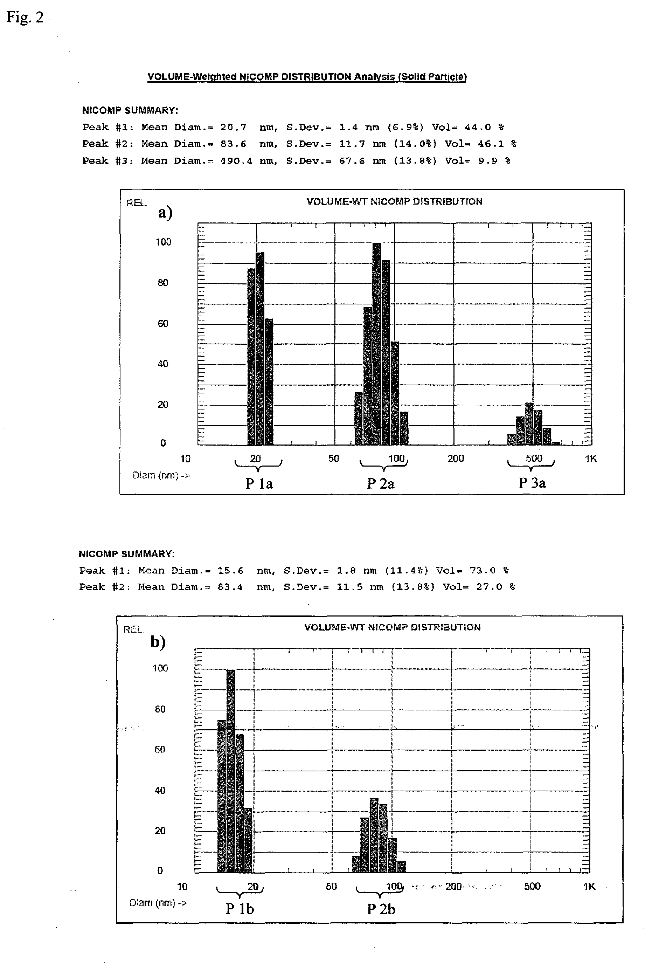

Fig. 2 is a diagram showing the particle size and particle size distribution measured by hydrodynamic laser scattering of an iron oxide nano-particle composition according to the invention which is coated with fatty acid and loaded with the cytostatic mitoxantron. Fig. 2a relates to the unfiltered sample and Fig. 2b relates to the samples being filtered with a 0.8 µm and a 0.22 µm filter.

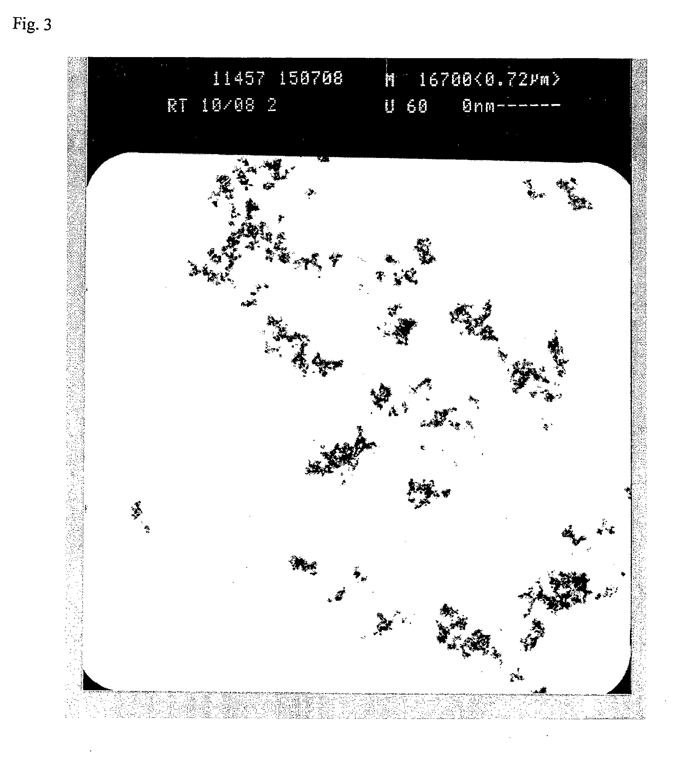

Fig. 3 is a TEM picture of an iron oxide nano-particle composition according to the invention which is coated with fatty acid and loaded with the cytostatic mitoxantron.

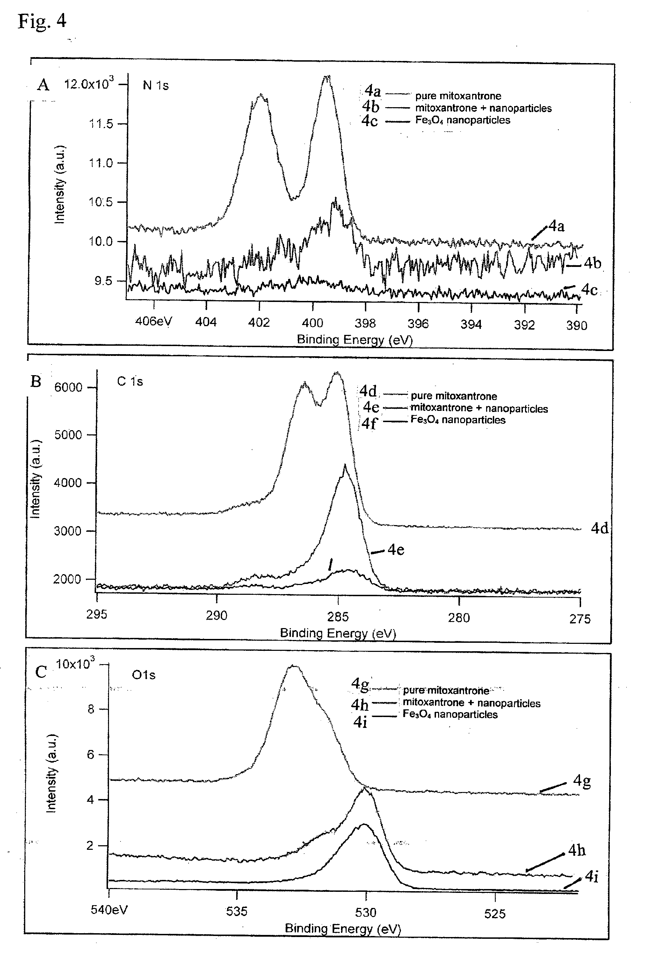

Fig. 4 illustrated a XPS-measurement verifying that the therapeutic agent (mitoxantron) is attached directly to the surface of the particle cores via a nitrogen atom by electrostatic interaction.

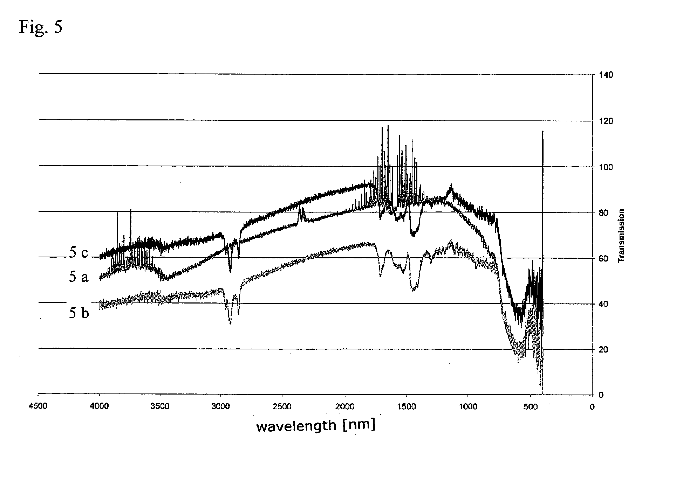

Fig. 5 illustrates DRIFT measurement curves obtained of raw nano-particles (5a), nano-particles coated with lauric acid (5b), and nano-particles coated with fatty acid and functionalized with mitoxantron (5c). The DRIFT measurements verify that the fatty acid is covalently bound to particle core.

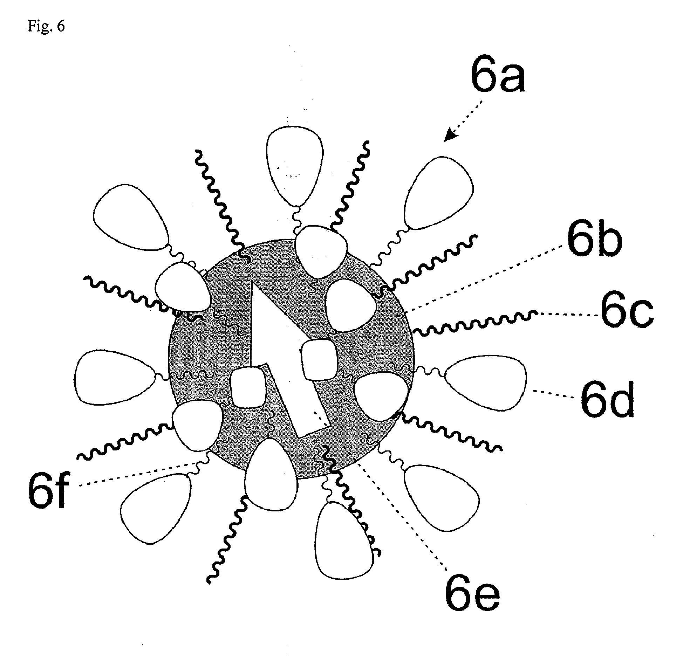

Fig. 6 schematically illustrates a nano-particle (6a) according to the invention, which comprises a particle core (6b) with a magnetic moment (6e) having a coating comprising a fatty acid (6c) and wherein a therapeutic agent (6d; e.g. mitoxantron) is adhered to the surface of the magnetic particle core via electrostatic interaction (6f).

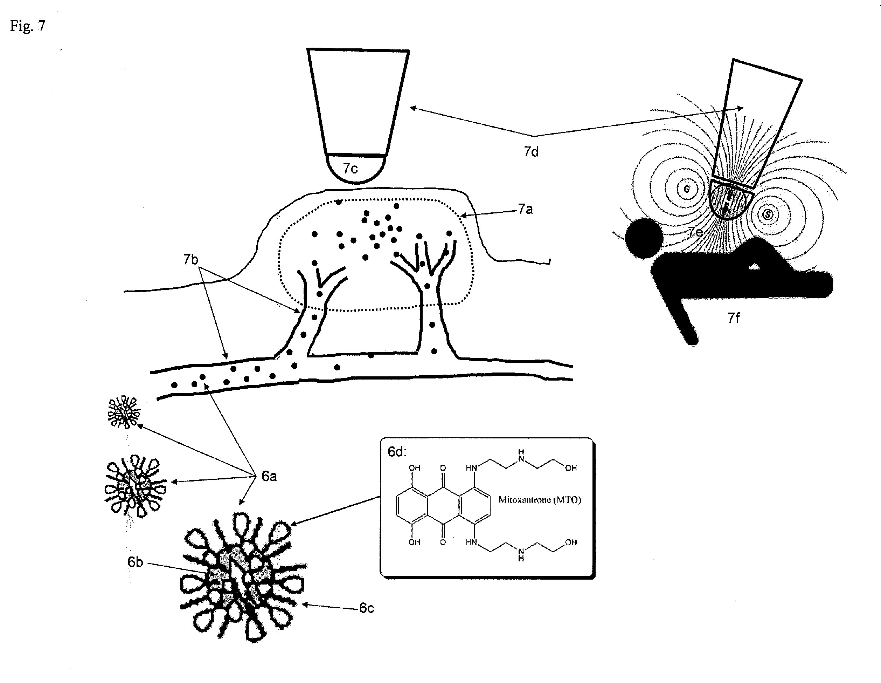

Fig. 7 schematically illustrates the concept of magnetic drug targeting taking advantage of the nano-particles (6a) according to the invention. The nano-particle composition (6a) is delivered to a tumor tissue (7a) of a patient (7f) through an artery (7b). The composition is guided and accumulated to the tumor tissue (7a) by a magnetic gradient field (7e) generated from the magnetic pole shoe (7c) of a magnet (7d).

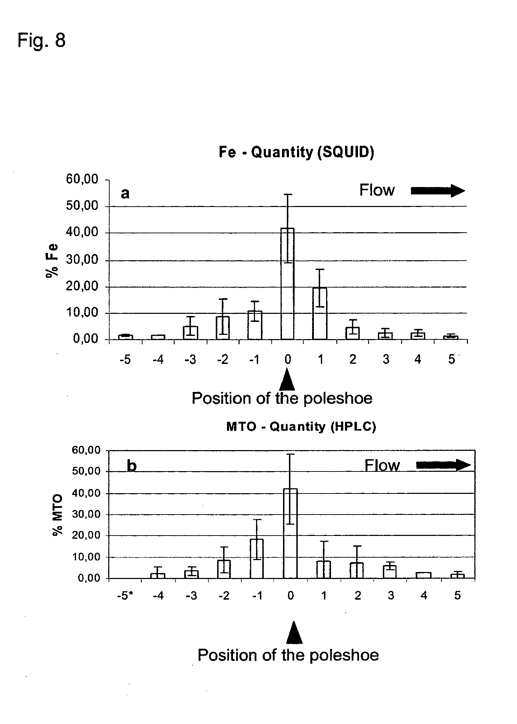

Fig. 8 illustrates an accumulation of the nano-particle composition according to the invention in different artery segments of an artery model under application of a magnetic gradient of 16 T/m at the pole shoe. Fig. 8a illustrates a Magnetorelaxometry (MRX)-measurement (SQUID) of the quantity of iron oxide particles. Fig. 8b illustrates a HPLC-measurement of the therapeutic agent mitoxantron attached to the particles.

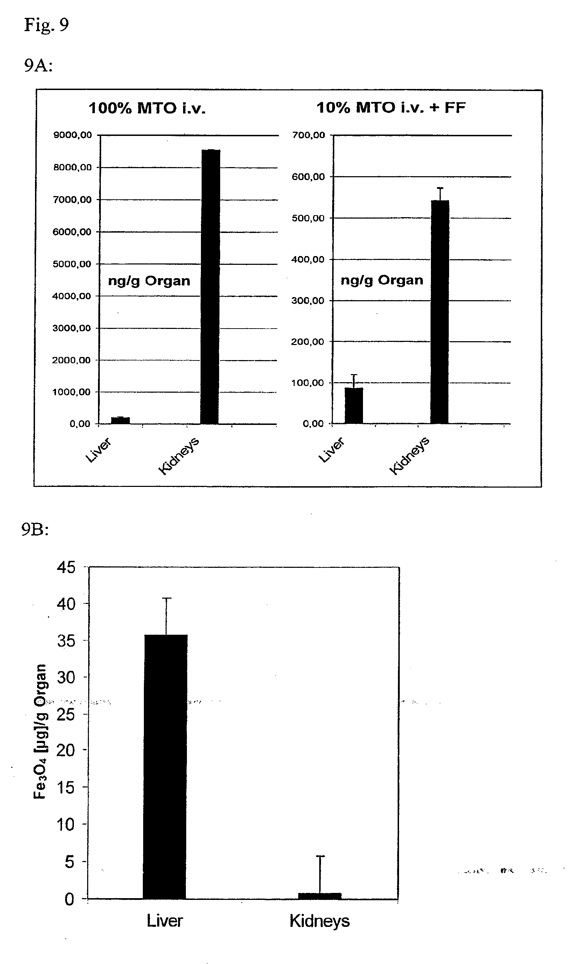

Fig. 9 illustrates the bio-distribution of MTO (Fig. 9A) and of iron oxide (Fig. 9B) in the liver and the kidneys in an animal model after intravenous application. Fig. 9A (left diagram) relates to an intravenous application (i.v.) of pure MTO (2.4-2.9 mg pure MTO; n=6) monitored by HPLC; Fig. 9A (right diagram) relates to an i.v. application of MTO attached to the nano-particles according to the invention containing 0.26-0.28 mg MTO (n=5) monitored by HPLC; Fig. 9B relates to the corresponding MRX-measurement (SQUID) monitoring the iron Fe₃O₄-amount in tissue after i.v. application of nanoparticle suspensions containing 0.26-0.28 mg MTO and 7.5-8 mg iron oxide respectively (n=5).

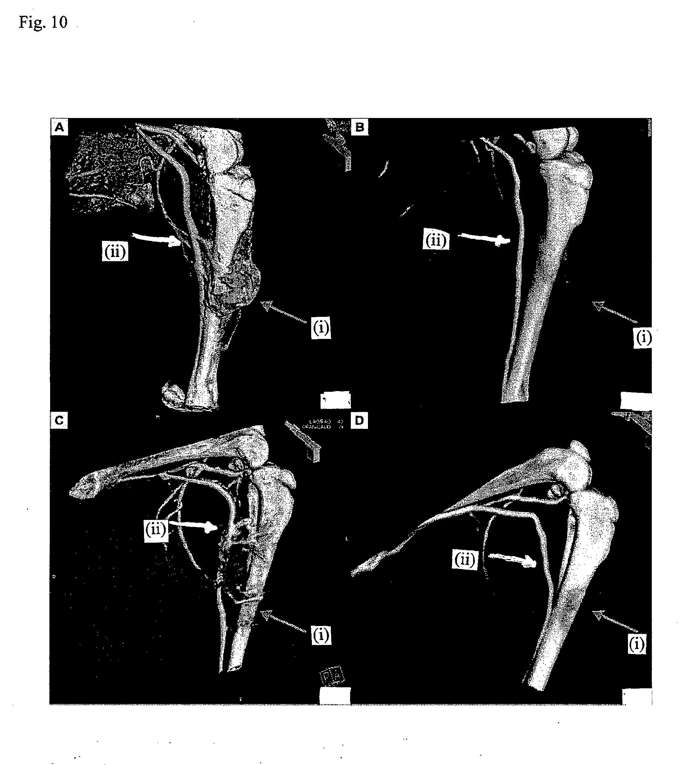

Fig. 10 relates to angiographic images illustrating the onset and progression of tumor remission after intra-arterial magnetic drug targeting (MDT) according to the invention, wherein the evaluation is carried out by means of Dyna-CT imaging technology. Figs. 11A & C: Dyna-CT before MDT. Figs. 11B & D: 7 weeks (animal A; Fig. 11B) and 5 weeks (animal B; Fig. D) after MDT. Arrows (i) indicate the tumor tissue. Arrows (ii) indicate the ateria femoralis with (Figs. 11A & C) and without (Figs. 11B & D) tumor vessels.

### Detailed Description of the Invention

According to a first aspect, the present invention relates to a pharmaceutical composition for magnetic targeting according to claim 1, wherein the composition comprises magnetic nano-particles, which comprise a magnetic iron oxide particle core and a coating comprising the fatty acid lauric acid.

Since the pharmaceutical composition of the present invention comprises magnetic nano-particles which can be guided and accumulated into a predetermined local area of a subject by application of an external magnetic field (magnetic targeting), there is no need to individually modify the nano-particles of the pharmaceutical composition of the present invention with any selective cell targeting means for each disease to be treated or imaged. Hence, the pharmaceutical composition according to the present invention gains access to application in therapy, since the pharmaceutical composition loaded with the therapeutic agent mitoxantrone can be delivered into a desired regional or local treatment area due to external guidance by a magnetic field.

The term "magnetic nano-particle" used in the present invention is not limited to a constitution comprising a single magnetic particle core but encompasses also an agglomerate of such particle cores.

Accordingly, the magnetic nano-particles comprised in the pharmaceutical composition of the present invention comprise a coating on the particle core and on an aggregate of particle cores. Such a coating is indispensible for *in vivo* applications of the magnetic nano-particles since it stabilizes the nano-particles, prevents formation of larger aggregates, while improving its biocompatibility.

The present inventors found that specifically a coating comprising the fatty acid not only stabilizes the nano-particles and prevents formation of larger aggregates, but, further, that such a coating also ensures that an agent (either an therapeutic agent, an imaging agent, or an mixture thereof) can be adhered directly onto the surface of the particle cores and/or into the inside of an aggregate of the particle cores by electrostatic interaction in a straight forward procedure in a high loading amount and in a very stable manner. Hence, according to the invention, there is no need for any further biocompatible coating on the particle cores in addition to the coating comprising the fatty acid.

In the present invention, above described fatty acid molecules are attached to the particle core by covalent binding as confirmed by DRIFT measurements. Hence, essentially all the fatty acid molecules are covalently attached the particle cores. However, it is also possible that not all of the fatty acid molecules are covalently bound to the particle core. In this case, a minor part of the fatty acid molecules can be associated to the particle core in a non-covalent manner. Fatty acid molecules that are not covalently bound to the particle core also contribute to the stabilization of a dispersant state of the pharmaceutical composition. It is assumed that the non-covalently associated fatty acid acts as a dispersant stabilizing the dispersion state of a pharmaceutical composition. The amount of fatty acid molecules that are not covalently attached to the particle cores may be up to 40%, preferably not more than 30%, more preferably not more than 25%, relative to the total amount of the fatty acid comprised in the coating.

By contrast to a polymer coating such as a resin coating, a starch coating or an dextran coating, a coating comprising the fatty acid does not cover the whole particle core surface or almost all of the particle core surface, but instead ensures that an agent gains access to the surface of the particle core and to the inside of an particle core aggregate. Hence, the coating of the present invention may optionally comprise other molecules, compounds, and materials, intended to further improve the stability, property, function, or specificity. It may be a molecule ensuring or improving the biocompatibility of the nano-particle or a molecule avoiding the nanoparticle to get trapped by the immune system. However, such further constituents in the coating are only optional and may only be present in an amount provided that the therapeutic agent can be adhered directly to the surface of the particle core and to the inside of an aggregate thereof.

The inventors surprisingly found that although the fatty acid coating according to the invention ensures that above agent gains access to the particle core surface contrary to known polymer coatings, the coating comprising the fatty acid also ensures that the above agent can be adhered to the particle core surface and to the inside of a particle core aggregate in a high amount and in a very stable manner.

The adherence is believed to be so strong since the aggregates of the particle cores need to be at least partially disintegrated in order to release the agent adhered therein. On the other hand, it is assumed that the fatty acid has some "shielding effect" preventing the agent to be released from the core surface. This might be due to the fact that the agent is not directly exposed on the surface of the coating but adhere to the surface of the particle core. Hence, the fatty acid is assumed to form a layer structure separating and shielding the agent adhered directly to the particle core surface from the surrounding environment such as an aqueous medium being present under physiological conditions.

Further, the inventors found that the coating comprising the fatty acid is stable even under autoclavation conditions. In other words, the pharmaceutical composition of the present invention is autoclavable without changing its characteristic attributes such as size, loading capacity with the agent and stability of the coated nano-particles of the invention. Therefore, the present invention for the first time describes a nano-particle composition which is applicable under clinical conditions since it can be obtained in a sterile and stable manner. The sterility of the pharmaceutical composition following autoclavation was confirmed by standard Endotoxin determination method evidencing that the stable composition was sterile even 14 days after incubation.

Therefore, according to one embodiment of this invention, the coating of the particle cores essentially consists of the fatty acid in a sense that it is ensured that an agent can be adhered directly to the surface of the particle core via electrostatic interaction. Accordingly, bulky polymers covering the surface of the particle core may not be comprised in such a coating.

As described above, additional constituents other than the fatty acid are only optional and must not to be comprised in the coating if they hamper to attain the above beneficial effects of the pharmaceutical composition according to the invention.

According to one embodiment, the coating of the particle cores preferably consists of the fatty acid. Apparently, such an embodiment has the benefit of being accessible in a straight forward manner without the need for additional modifications.

Further, the magnetic material constituting the particle core is pharmacologically acceptable, can be coated with the fatty acid, and is able to adhere the above mentioned agent via electrostatic interaction. The material for forming the particle core is an iron oxide, which may be ferromagnetic, ferrimagnetic or super paramagnetic. It may be a mono-or multidomain material. Preferably, it is a mixed mono- and multidomain material. Examples include compounds such as MO-Fe₂O₃ and M-Fe₂O₄, wherein M may be represented by Mg, Al, Si, Ca, Sc, Ti, V, Fe, Co, Ni, Cu, Zn, Sr, Zr, Nb, Mo, Sn, Ba or Li, either alone or in combination. Preferably, the magnetic core particles may be Fe₂O₃ and/or Fe₃O₄.

In particular, a mixed mono- and multidomain Fe₂O₃/Fe₃O₄ iron oxide core shows high responsiveness and accumulation properties in magnetic targeting applications and therefore represent a preferred embodiment of this invention.

The fatty acid comprised in the coating of the pharmaceutical composition of the present invention is lauric acid in order to achieve best results concerning the stability of the dispersion state of the pharmaceutical composition, of the loading capacity with an agent and the stability of the adherence of this agent to the particle core surface.

According to a specifically preferred embodiment, the pharmaceutical composition comprises above described Fe₂O₃/Fe₃O₄ particle cores being coated with lauric acid. Such an embodiment is accessible in a straight forward manner and provides favorable magnetic and loading characteristics.

According to the invention, above particle cores are coated with 25 to 45 parts by weight of the above described fatty acid relative to 100 parts per weight of the particle core. In order to balance the loading capacity and the stability of the loading it is preferred to coat the particle cores with 25 to 35 parts per weight of the fatty acid relative to 100 parts per weight of the particle core.

The pharmaceutical composition of the invention preferably has a Zeta-potential in an aqueous dispersion medium of from -15 to -45 mV. Having a Zeta-potential in this range the dispersion state of the pharmaceutical composition according to the invention is stabilized. This effect is even more improved for above described magnetic particles exhibiting a Zeta-potential in the range of from -20 to -35 mV.

The pharmaceutical nano-particle composition can be functionalized with an agent in a straight forward manner. In any case, it is essential for the present invention that the particle cores comprise the fatty acid coating since otherwise a stable adherence of the agent in a sufficient amount cannot be accomplished.

The above described magnetic particles further comprise the agent mitoxantrone, which is adhered directly onto the surface of the particle core and to the inside of a particle core aggregate via electrostatic interaction. From the viewpoint of ensuring the adherence by electrostatic interaction in a stable manner with a high amount, this agent is constituted by an aromatic skeleton comprising at least a hetero atom.

According to a specifically efficient embodiment as illustrated in Fig. 6, the pharmaceutical composition comprises the above described Fe₂O₃/Fe₃O₄ particle cores, which are coated with lauric acid and wherein mitoxantron is adhered to the particle core surface.

According to the invention, the above agent is preferably loaded with an amount of at least 0.1 parts per weight relative to 100 parts per weight of the particle core. More efficiently, the particles are loaded with at least 1 part per weight and more preferably with at least 2 parts per weight of the agent relative to 100 parts per weight of the particle core. It is preferred to load as much of the agent to the particle cores as possible to reduce the overall amount of the pharmaceutical composition to be administered.

The pharmaceutical composition of the invention further comprises a dispersion medium to give a so-called ferrofluid. This medium is an aqueous medium. This aqueous medium may include buffering agent, salts and dispersants under the premise that those constituents are pharmaceutically acceptable. By being provided in the form of a ferrofluid, the pharmaceutical composition of the invention can be administered very efficiently and is well tolerated.

The dispersion has a solids content ranging from 8 to 25 mg/ml. A solids content in the above range is well tolerated by a subject being treated with the composition.

The pharmaceutical composition preferably comprises magnetic particles having a particle size of less than 220 nm as measured by hydrodynamic laser scattering in a mono- or multi-dispersed state in an aqueous medium. If the particle size is more than 220 nm, there is a danger that the particles are accumulated in the liver and the spleen and the formation of larger aggregated leading to embolization is avoided. It is preferred that the magnetic particles have a particle size of less than 180 nm, or even below 140 nm. By exhibiting a particle size below such ranges, the magnetic particles of the present invention have further improved diffusion properties in the tissue. On the other hand, it is preferred that the magnetic particles of the composition may have a particle size of at least 5 nm, preferably more than 10 nm, in order to ensure that the magnetic particles properly respond to an external magnetic field.

Apparently, the pharmaceutical composition according to the invention has the benefit to be designed for therapeutic indications specifically for regional and local diseases.

In another aspect, the present invention relates to a pharmaceutical composition carrying the therapeutic agent as described above for use as a medicament as first medical indication.

In still another aspect, the present invention relates to a pharmaceutical composition as described above for use in cancer treatment by magnetic drug targeting using an external magnetic field as a second medical indication. The pharmaceutical composition of the invention is specially suited for carrying high toxic agents to a local disease area by taking advantage of magnetic properties of the particle composition in response to an external magnetic field.

The pharmaceutical composition for use in cancer treatment by magnetic drug targeting may, depending on the kind of target tissue and nature of drug, be administered in a therapeutically effective amount by any known methods including any injection methods. However, the present inventors found that an intra-arterial application of the pharmaceutical composition is especially favorable. If administering the pharmaceutical composition intra-arterial, it is possible to prevent that metabolic barriers interfere with an effective accumulation of the nano-particles in the target tissue. Also the danger of nano-particle induced thrombosis is avoided by intra-arterial application.

Furthermore, it is preferred to determine the positioning and the vascularization of the target tissue and the position for accessing the arterial system by angiographic imaging, e.g. by Dyna-CT, before the application of the composition. Following the determination for accessing the arterial system, the pharmaceutical composition can be administered by catheter technology ensuring that the injected pharmaceutical nano-particle composition can be accumulated in the target tissue by means of an external magnetic field in a very efficient manner. Accordingly, only a fractional amount of the standard systemic dosage is necessary to achieve even better therapeutic result.

The pharmaceutical composition for use in a cancer treatment by magnetic drug targeting using an external magnetic field is preferably administered in a therapeutically effective amount being adjusted by means of online monitoring the target tissue. Hence, if the pharmaceutical composition contains a monitoring agent it is possible to image the accumulation of the composition in the target tissue and stop the continued supply of the pharmaceutical composition if no further accumulation can be recognized. Hence, it is possible to avoid administering excessive amount of the pharmaceutical composition.

The composition may be administered by one time administration in case this results in the desired therapeutic success. However, in case a repeated administration turns out to be necessary, as might be indicated by angiographic and MRT-based monitoring, the administration may be repeated as many times as necessary. By successively monitoring the onset and success of therapy, it is possible to avoid administering successive amount of toxic agents preventing undesired side effects.

When being used for cancer treatment by magnetic drug targeting, the pharmaceutical composition of the invention is preferably guided and accumulated to the target tissue by application of an external magnetic field gradient. In order to attain best accumulation results, it is preferred that the magnetic gradient field has a high magnetic flux density ranging from 0.5 to 1.5 T, preferably around 1 T, and a magnetic field gradient ranging from 10 to 90 T/m, preferably above 30 T/m or even above 60 T/m.

Preferably, the pharmaceutical composition is guided and accumulated in the target tissue by a multi directional magnetic field being spatially variable, preferably by means of different pole shoes. When being spatially variable, the present invention is able to accurately guide and accumulate the pharmaceutical composition to the target tissue of various form of appearance in a very precise manner.

Apparently, above described method steps are not limited to the treatment of cancer as described above but may also be applied for other regional or local diseases such as arteriosclerosis, rheumatoid arthritis, inflammations (e.g. osteomyelitis).

According to a further aspect, the present invention relates to a method for manufacturing the above described pharmaceutical composition. The method comprises at least the steps of producing magnetic iron oxide nano-core particles, coating the particle cores with the fatty acid lauric acid and loading mitoxantrone onto the surface of the particle cores and/or the inside of an aggregate of the particles cores by electrostatic interaction.

The manufacturing method of the magnetic nano-particle cores as described above is not especially limited and multiple manufacturing procedures have been described in literature. From the viewpoint of an easy and reliable manufacturing procedure of the nano-particle cores being especially suited for the present invention, it is preferred that the above described iron oxide particles are precipitated from an aqueous solution containing iron(II) and iron(III) salts by addition of a base, which may be an ammonia solution. This ensures to obtain iron oxide particles in a favorable size and aggregation state in a straight forward and reliable manner.

As the iron(II) and iron(III) salts, especially suited are iron chlorides or sulfates. Preferably, the iron(II) and iron(III) salts are provided at a molar ratio of 2:3. By adjusting this specific molar ratio, the present invention is able to produce mono- and multi-domain Fe₂O₃/Fe₃O₄ core particles having favorable magnetic properties in a reliable manner.

The thus produced magnetic nano-particle cores are subsequently coated with the fatty acid. This is preferably accomplished under elevated temperature conditions to ensure that of the fatty acid molecules are covalently bound. Therefore, the nano-particle cores are preferably suspended, particularly preferably in an aqueous medium, and the temperature is preferably elevated to 60°C or more, preferably 90°C or more. The elevated temperature is preferably held constant for several minutes, usually between 2 to 20 minutes. Then, the suspension is allowed to slowly cool to room temperature. The coating procedure may be a one time procedure or it may be repeated once or more times. By repeating the coating procedure at least once, it is possible to increase the coating amount of the fatty acid, and, thereby, subsequently also increase the coating amount of the above described agent.

According to a specific embodiment, the above described mono- and multi-domain Fe₂O₃/Fe₃O₄ core particles are coated with lauric acid, while preferably repeating the coating procedure once.

The manufacturing method of the invention preferably further comprises a step of autoclavation of the composition. From the viewpoint of *in vivo* application, it is highly preferred or even required to autoclave the pharmaceutical composition so as to satisfy high demands on sterility. The pharmaceutical composition according to the invention has the advantage that the coating comprising a fatty acid is stable even under autoclavation conditions commonly applied (e.g. 120°C, 1 bar over pressure, 20 min). This means that the characteristics of the pharmaceutical composition do not change by this autoclavation process. By contrast to prior art, for instance, polymer starch coatings, the nano-particles of the pharmaceutical composition of the invention having a coating comprising the fatty acid retain their essential characteristics. Hence, the nano-particles substantially retain their size, the Zeta-potential, and the loading capacity for the above described agents, if being sterilized by an autoclavation procedure. The autoclavation is preferably applied after the coating step of the particle cores with the fatty acid and before the loading with the above agent.

The manufacturing method of the pharmaceutical composition comprises the step of loading the nano-particles, which comprise the fatty acid coating, with the agent mitoxantrone comprising an aromatic skeleton and a hetero atom. Said agent is adhered directly onto the surface of the particle cores and/or into the inside of an aggregate of the particle cores by electrostatic interaction.

The loading step preferably comprises diluting the coated magnetic core particles to obtain a dispersion state, homogenizing the dispersion and adding said agent at ambient temperature so as to achieve stable adhesion with a high amount. The dispersion may preferably be an aqueous dispersion. Further, the homogenization may be made with the aid of any suitable conventional means, but it is preferred to homogenize the dispersion by ultrasonic treatment from the viewpoint of straight forward synthesis. The temperature for the loading step is not particularly limited, but it may not exceed a temperature which conflicts with a stability of the agent. It is sufficient to add the agent at ambient temperature and the temperature may therefore range from 10 to 35°C.

The method for manufacturing the pharmaceutical composition as described above preferably further comprises the method of separating the nano-particles so as to eliminate particles and particle agglomerates having a size of 220 nm or more. Preferably, the separating step eliminates particles and particle agglomerates having a size of 180 nm or more. The method of separating is not especially limited but may preferably be accomplished by filtration, since a filtration does not require expensive separating means. For example, the nano-particle composition may be filtered through a 0.8 µm syringe filter and a 0.22 µm syringe filter. The filtration is conventionally performed after the loading with the above described agent so that the filtered pharmaceutical composition is ready for the subsequent application into a subject to be treated. However, in addition to this final filtration step, there may for instance also be performed a filtration step prior to the coating step with the above agent.

A manufacturing method of the pharmaceutical nano-particle composition is also schematically illustrated in figure 1.

The present description provides a method for guiding and accumulating the above described pharmaceutical composition being contained in a subject to a target tissue of this subject by means of an external magnetic field gradient. The magnetic field gradient has preferably a high magnetic flux density ranging from 0.5 to 1.5 T, preferably around 1 T, and a magnetic field gradient ranging from 10 to 90 T/m, preferably above 30 T/m or even above 60 T/m.

By means of such magnetic field parameters, it is possible to precisely accumulate the pharmaceutical nano-particles composition of the invention to a target tissue of any shape. Hence, compared to for instance conventional permanent magnet based methods the invention can increase the accumulation of the composition in a local area of any shape and size. This method enables to therapeutically treat a target tissue. The method thus represents a therapy which is suitable for any regional or local target tissue of any subject. Hence, the target tissue to be monitored or treated is not specifically limited and the method is suited for any regional or local disease such as arteriosclerosis, rheumatoid arthritis, inflammations.

Preferably, the target tissue in the method for guiding and accumulating the pharmaceutical composition is a tumor. Hence, it is possible to therapeutically treat various kinds of cancer of a patient.

The method for guiding and accumulating as described above is very efficient if taking advantage of a magnetic field which is a multidirectional magnetic field being especially variable by means of different potions. By this constitution, it is possible to guide and accumulate the composition in a very precise and effective manner.

In the above described method, the pharmaceutical composition is administered to the subject in a therapeutically effective amount by any known methods including various injection methods. The effective amount depends on the kind of target tissue, its size and weight, as well as the size and weight of the subject to be treated. Of course, the effective amount also depends on the agent to be administered in a sense of its efficacy and toxicity. As described above, it is preferred to intra-arterially administer the composition from the viewpoint of lowering the effective amount of the pharmaceutical composition while preventing side effects. For intra-arterial administration, preferably catheter technology is used. The composition may be administered up to an amount still being tolerated by the treated subject.

Preferably, the method is performed such that the amount of the pharmaceutical composition to be administered is adjusted by means of online monitoring the target tissue. Thereby, the accumulation of the pharmaceutical composition at the target tissue is evaluated in real time. Hence, if the online monitoring reveals that no further accumulation of the pharmaceutical composition is achieved, the administration can be stopped to avoid applying excessive amounts of the pharmaceutical composition. Further, the composition may be administered only once if this is sufficient for the onset and progression of therapeutic success, i.e. tumor remission. However, the administration may also be repeated preferably following angiographic or MRT-based monitoring revealing that a continued therapy is necessary for the desired therapeutic success.

The above described method is especially effective if taking advantage of determining the positioning and the vascularization of the target tissue and the position for accessing the arterial system, which can be accomplished by angiographic imaging, e.g. by Dyna-CT. As previously described, following such a determination treatment the pharmaceutical composition of the present invention can be administered in a very precise manner to a designated application area thereby ensuring that the greatest possible proportion of the pharmaceutical composition actually reaches the target tissue.

The pharmaceutical composition is preferably administered by catheter technology ensuring that the injected pharmaceutical nano-particle composition can subsequently be accumulated into the target tissue by means of an external magnetic field in a very efficient manner. As a result, only a fractional amount of the standard systemic dosage is necessary to achieve the desired therapeutic success.

The present invention will subsequently be described in detail based on non-limiting examples.

### Examples

The nano-particles and the composition of the present invention are characterized and determined according to the following procedures.

### Hydrodynamic layer scattering

Hydrodynamic Diameter: Dynamic laser scattering (DLS, Nicomp 380 ZLS, Santa Barbara, CA, USA) measurements of the hydrodynamic diameter of the particles. Size distribution due to volume weighted nicomp distribution analysis (solid particles).

### Detailed Parameters:

Temperature: 23°C
Liquid viscosity: 0.933 CP
Liquid Indey of Refraction: 1.33
Intensity Setpoint: 300 kHz
External Fiber Angle: 90°
Scattering Angle: 90°

### Zeta-potential

The Zeta-potential is measured using the same apparatus as described for the hydrodynamic diameter measurements but in the Zeta-potential modus.

### Detailed Parameters:

Temperature: 23°C
Liquid viscosity: 0.933 CP
Liquid Indey of Refraction: 1.33
Intensity Setpoint: 300 kHz
External Fiber Angle: 18.9°
Scattering Angle: 14.06°
Dielectric Constant: 78.5
Electrode Spacing: 0.4 cm
Electric Field Strength: 10.5V/cm

### Autoclavation

### H+P Varioklav E 400 EP-Z

### Conditions:

120°C, 1 bar over pressure, 20 min

### DRIFT-measurement

Varian Excalibur FTS 3100 spectrometer with a resolution of 4 cm⁻¹.

### Example 1

16.12 g FeCl₂ * 4H₂O and 32.33 g FeCl₃ * 6H₂O (molar ratio of 2:3) are dissolved in 133 ml water. Under stirring 25% NH₃-solution is added to precipitate Fe₂O₃/Fe₃O₄ particles. Then, the iron oxide precipitate is caused to sediment by storage above a permanent magnet and the chloride is removed by washing the iron oxide precipitate with an aqueous solution containing 1.3% NH₃. Subsequently, the washed iron oxide is taken up in 200 ml 1.3% NH₃ solution and an aliquot of 20 ml of this raw ferrofluid is mixed with 2.7 mmol lauric acid and heated to 90°C under stirring. The temperature is held constant at 90°C for 4 minutes and the mixture is subsequently allowed to cool to room temperature (AT = 1°C/min). Then, the ferrofluid is diluted with water to a volume 8.5 times the starting volume followed by autoclavation. An aliquot of this diluted ferrofluid (5 ml) is homogenized by ultrasonic treatment and 350 µl mitoxantron (MTO; content 2mg/ml) is added to the ferrofluid. Thereafter, the functionalized ferrofluid is filtered through a 0.8 µm syringe filter and, subsequently, through a 0.22 µm syringe filter. The thus obtained ferrofluid is ready for application and has the following characteristics:

### Solids content:

10.92 mg/ml

### Mass fractions of solids content:

Fatty acid: 28.8%; iron oxide core: 70.0%; MTO: 1.2%

### Particle size:

The particle size of the ferrofluid has been characterized by laser scattering before and after the final filtration step as illustrated in figure 2 (the x-axis donated the particle diameter in nm and the y-axis donated the relative intensity of the peak). As apparent from figure 2a, the unfiltered ferrofluid contains three peak fractions of different sizes, namely Pla, P2a, and P3a. Concretely, the peak fraction P1a has a mean diameter of around 20.7 nm (with a standard deviation of 1.4 nm (6.9%)) in an amount of 44.0% per volume), the peak fraction P2a has a mean particle diameter of 83.6 nm (with a standard deviation of 11.7 nm (14.0%)) in an amount of 46.1% per volume, and the third peak fraction 3Pa has a mean particle diameter of 490.4 nm (with a standard deviation of 67.6 nm (13.8%)) in an amount of 9.9% per volume. After filtration (figure 2b), the ferrofluid contains two peak fractions 1Pb and 2Pb. Concretely, the peak fraction 1Pb has a mean particle diameter of 15.6 nm (with a standard deviation of 1.8 nm (11.4%)) in an amount of 73.0% per volume and the peak fraction 2Pb has a mean particle diameter of 83.4 nm (with a standard deviation of 11.5 nm (13.8%)) in an amount of 27.0% per volume.

### Zeta-potential:

The filtered ferrofluid has a Zeta-potential in the range from -25 to -30 mV.

### Particle morphology:

As illustrated in figure 3, an image obtained by transmission electron microscopy (TEM) reveals that the mono and multi-dispersed particles having a maximum size of around 100 nm.

### Binding mode of fatty acid and therapeutic agent:

The ferrofluid has been investigated by a high-resolution X-ray measurement (x-ray photoelectron spectroscopy, XPS) to confirm that the therapeutic agent is adhered directly onto the surface of the particle cores and/or into the inside of an aggregate of the particle cores by electrostatic interaction. An exemplary XPS spectrum is illustrated in Figures 4A to 4C. Fig 4A shows the N 1s signal curves for pure mitoxantron (4a), mitoxantron + nanoparticle (4b), and the Fe₃O₄ nanoparticle (4c). Fig 4B shows the C 1s signal curves for pure mitoxantron (4d), mitoxantron + nanoparticle (4e), and the Fe₃O₄ nanoparticle (4f). Fig 4C shows the O 1s signal curves for pure mitoxantron (4g), mitoxantron + nanoparticle (4h), and the Fe₃O₄ nanoparticle (4i). The XPS measurement thus confirms the non covalent binding of mitoxantron via the nitrogen atom to the surface of the nanoparticle. The ferrofluid was further investigated by a DRIFT measurement to confirm that the fatty acid is attached to the particle core via covalent binding. Fig. 5 shows the spectrum of pure ferrofluid (5a), whereas spectrum 5b shows ferrofluid and lauric acid. The absorption band at 1450-1500 nm indicates the O-H bond, the absorption band at 1700 nm the C=O bond, and the absorption band at 2900 nm the C-H of lauric acid. Apparently, curve 5c relating to ferrofluid + lauric acid + MTO is congruent with the spectrum of 5b, no further absorptions bands are present evidencing that MTO has no covalent binding content.

Furthermore, the supernatant solution was investigated by means of HPLC measurements confirming that the supernatant solution contained less than 8wt.-% of the added mitoxantron evidencing that almost all of the mitoxantron is attached to the nano-particles.

As a result, a nano-particle of this Example has a constitution as illustrated in figure 6.

### Stability:

The stability of the pharmaceutical nano-particle composition was investigated in different media to determine the release amount of the mitoxantron dependent on the pH. The table illustrated the release of MTO in buffered Ringer solution after 60 minutes for the ferrofluid of this example and Dextran-coated reference particles.

| pH | release [%] | Release of Dextran-coated reference particles |
|---|---|---|
| 7,172 | 1,6 | 31,7 |
| 5,945 | 1,6 | 42,2 |
| 5,403 | 1,8 | 61,9 |
| 4,146 | 3,7 | 86,3 |
| 3,13 | 37,7 | 87,9 |

Apparently, prior art particles such as Cysteine- (data not shown) and Dextran-coated particle release much more mitoxantron compared to the magnetic nano-particles of this invention comprising a fatty acid coating.

### Sterility

The sterility of the pharmaceutical composition following autoclavation was confirmed by standard Endotoxin determination method evidencing that the stable composition was sterile even 14 days after incubation.

### Ex vivo artery model:

The response properties of the pharmaceutical composition to an external magnet field were investigated in an ex *vivo* artery model. It is demonstrated that the pharmaceutical composition accumulates in different artery segments of an artery model under application of a magnetic gradient of 16 T/m at the pole shoe. Fig. 8a illustrates a Magnetorelaxometry (MRX)-measurement (SQUID) of the quantity of the iron oxide particles. Fig. 8b illustrates a HPLC-measurement of the therapeutic agent mitoxantron attached to the iron oxide particles. As apparent from figure 8, the nano-particles of Example 1 accumulate in the artery segments having the shortest distance to the pole shoe of the electro magnet and thus having the strongest influence of the magnetic field. In Fig. 8, the X-axis donates the position of the pole shoe relative to the artery and in flow direction of the buffer circulation. 0 indicated the position underneath the tip of the pole shoe, whereas -1 and +1 indicate a segment in relative position before respectively after the pole shoe.

### Biodistribution and biodegradation:

For the animal experiments New Zealand White Rabbits were used. The animals, weighing about 4.5 - 5.2 kg, were treated (i.v.) with ferrofluid bound MTO containing a drug amount of 10% compared to the regular systemic dose which requires 10 mg MTO/m2 body surface (n=5). The other study group (n=6) received the regular systemic dose of pure MTO. After 24h hours the animals were sacrificed and the organs harvested. Complete organs of liver and kidneys were processed and MTO was extracted for HPLC-analysis [4]. Tissue homogenates have also been measured by SQUID.

### Results

HPLC-measurements show a high enrichment of MTO in kidneys concerning both application modes (Fig. 9). Nanoparticle mediated drug application dramatically influences the biodistribution of MTO after i.v. injection. Application of pure MTO (Fig. 9A, left diagram) leads to 190 ng/g in liver tissue and 8500 ng/g in kidneys. A nanoparticle guided administration of MTO (Fig. 9A, right diagram) leads to a MTO concentration of 86 ng/g in liver and 540 ng/g in kidneys. With this application mode, MTO enrichment in kidneys is only 6-fold higher than in liver compared to a 44-fold increased accumulation after pure i.v. injection. The shift in the relation of MTO enrichment between liver and kidneys is due to the reticulo-endothelial-system (RES) trapping unsolved particles from the blood stream. Further, according to figure 9B, the RES leads to an enrichment of the magnetic nanoparticles in the liver, confirmed by SQUID (MRX) analysis of iron oxide detecting more than 35 µg/g iron oxide in liver, but only traces of less than 1 µg/g in kidneys. The amount of MTO, however, is not proportional to the detected iron in the liver, which leads to the suspicion, that MTO is released from the particles during its degradation.

### Example 2

The nano-particle composition of example 1 was used for a tumor therapy by magnetic drug targeting under the following conditions.

### Application:

Magnetic field: voltage: 30 V; A: 70 A
1 T field with a gradient of 72 T/m at the pole shoe

### Animal A

Rabbit with VX 2 Squamous Cell Carcinoma
weight: 3.9 kg
applied dosage: 1.5 ml ferrofluid;
amount of mitoxantron (MTO) = 0.198 mg, which corresponds to 8.3 % of the conventional systemic dosage.

### Animal B

Rabbit with VX 2 Squamous Cell Carcinoma
weight: 3.15 kg
applied dosage : 1.56 ml ferrofluid
amount of mitoxantron (MTO) = 0.22 mg, which corresponds to 10.5 % of the conventional systemic dosage.

In both animals the ferrofluid of Example 1 is intra-arterially with an intravenous (IV) cannula over 10 min. The magnetic field is subsequently applied for 5 min following the injection.

A third animal has been treated with a 1:500 dilution of the ferrofluid for MRT imaging the biodistribution in this rabbit. The MRT imaging confirmed that the ferrofluid is efficiently accumulated in the targeted tumor in an efficient manner (data not shown).

As illustrated in figure 11, an angiographic imaging by Dyna-CT visualizes that this therapy leads to a complete tumor remission following only one application of the pharmaceutical nano-particle composition (ferrofluid) of example 1 in a dosage corresponding to 7-10% of the conventional systemic dosage.

### Example 3

The manufacturing procedure of Example 1 is repeated. However, 40ml aliquot of the raw ferrofluid was treated by ultrasonic treatment (Bandelin Sonorex Digitec) without application of a temperature treatment. Then 1.066 g lauric acid was added, the mixture was heated to 90°C while stirring and holding the temperature for 4 min. Thereafter, the suspension was slowly cooled and the coating procedure was repeated once (1.066 g lauric acid, 90°C for 4 min. Then, the suspension was allowed to slowly cool to room temperature under continued stirring. Thereafter, the suspension was diluted with water to 8.5 times the volume (170 ml). After autoclavation, 6 ml of the ferrofluid being coated with lauric acid twice was homogenized in an ultrasonic bath. 5.0 ml of the coated ferrofluid was placed in a 50 ml vessel, was agitated by a Vortexer. Under agitation 350µl Mitoxantron solution (ready for use pharmaceutical, 2mg/ml) was added dropwise.

The resultant ferrofluid was characterized as described for Example 1 above.

The ferrofluid of Example 3 thus characterized had a solids content of 10.92 mg/ml. The mass fractions of the solids content were 30.25% fatty acid, 68.50% iron oxide core, and 1.25% MTO.

Further, the filtered ferrofluid had a Zeta-potential in the range from -25 to -30 mV and the mono and multi-dispersed particles had a maximum size of around 100 nm (a first peak fraction having a mean diameter of around 11.0 nm with a standard deviation of 0.7 nm in an amount of 86.2% per volume, and an second peak fraction having a mean particle diameter of 80.2 nm with a standard deviation of 9.1 nm in an amount of 13.8% per volume). Further, the supernatant solution of the ferrofluid contained less than 8wt.-% of the added mitoxantron.

### Conclusion

The present invention synergistically combines the above described pharmaceutical composition and the above described specifically adapted method for guiding and accumulation this composition to a target tissue. While being suited for regional and local diseases of various kinds, it is apparent that the pharmaceutical composition and the method for its accumulation make full use of their capabilities in the treatment of tumors.

## Claims

1. A pharmaceutical composition suitable for magnetic targeting, the composition comprising magnetic nano-particles, an aqueous dispersion medium having a solids content ranging from 8 to 25 mg/ml, the particles comprising a magnetic particle core and a coating comprising a fatty acid, wherein the fatty acid is lauric acid, and wherein the particle cores are coated with 25 to 45 parts by weight of the fatty acid relative to 100 parts by weight of the particle core, wherein the particle core is an iron oxide, and wherein the magnetic particles further comprise mitoxantrone, which is adhered directly onto the surface of the particle cores and/or into the inside of an aggregate of the particle cores by electrostatic interaction.

2. A pharmaceutical composition according to claim 1, wherein the particle core is a mono- and multi-domain Fe₂O₃/Fe₃O₄.

3. A pharmaceutical composition according to claim 1 or 2, wherein the magnetic particles have a particle size of less than 220 nm as measured by dynamic light scattering in a mono- or multi-dispersed state in an aqueous medium.

4. A pharmaceutical composition according to any of claims 1 to 3 for use as a medicament.

5. A pharmaceutical composition according to any of claims 1 to 3, for use in cancer treatment by magnetic drug targeting using an external magnetic field.

6. A pharmaceutical composition for use according to claim 5, wherein the composition is administered intra-arterial in a therapeutically effective amount, and/or wherein the positioning and the vascularization of the target tissue and the positioning for accessing the arterial system are determined by angiographic imaging.

7. A pharmaceutical composition for use according to claim 5 or 6, wherein the amount of the pharmaceutical composition to be administered is adjusted by means of on-line monitoring the target tissue and/or wherein the composition is administered by one-time administration or by repeated administration following angiographic and MRT-based monitoring.

8. A pharmaceutical composition for use according to any of claims 5 to 7, wherein the pharmaceutical composition is guided and accumulated to the target tissue by application of an external magnetic field gradient having a high magnetic flux density ranging from 0.5 to 1.5 T and a magnetic field gradient ranging from 10 to 90 T/m.

9. Method for manufacturing a pharmaceutical composition according to any of claims 1 to 8, the method comprising at least the steps:
- producing magnetic iron oxide nano-particles as said particle cores,
- coating said particle cores with lauric acid at least once, and
- loading mitoxantrone onto the surface of the particle cores and/or into the inside of an aggregate of the particle cores by electrostatic interaction.

10. Method for manufacturing a pharmaceutical composition according to claim 9, wherein the nano-particles are iron oxide particles which are precipitated in an aqueous solution containing iron-(II) and iron-(III) salts by adding a base.

11. Method for manufacturing a pharmaceutical composition according to claim 9 or 10, wherein the method further comprises a step of autoclavation of the composition.

12. Method for manufacturing a pharmaceutical composition according to any of claims 9 to 11, wherein the method further comprises the step of separating the nano-particles so as to eliminate particles and/or particle agglomerates having a size of 220 nm or more.

## Patentansprüche

1. Pharmazeutische Zusammensetzung, die zum magnetischen Targeting geeignet ist, wobei die Zusammensetzung magnetische Nanoteilchen, ein wässriges Dispersionsmedium mit einem Feststoffgehalt im Bereich von 8 bis 25 mg/ml, umfasst, wobei die Teilchen einen magnetischen Teilchenkern und eine Fettsäure umfassende Beschichtung umfasst, wobei die Fettsäure Laurinsäure ist, und wobei die Teilchenkerne mit 25 bis 45 Gewichtsteilen der Fettsäure relativ zu 100 Gewichtsteilen des Teilchenkerns beschichtet sind, wobei der Teilchenkern ein Eisenoxid ist, und wobei die magnetischen Teile ferner Mitoxantron umfassen, welches direkt auf der Oberfläche der Teilchenkerne und/oder in das Innere eines Aggregats der Teilchenkerne durch elektrostatische Wechselwirkung angehaftet ist.

2. Pharmazeutische Zusammensetzung nach Anspruch 1, wobei der Teilchenkern ein Mono- und Multi-Domainen Fe₂O₃/Fe₃O₄ ist.

3. Pharmazeutische Zusammensetzung nach Anspruch 1 oder 2, wobei die magnetischen Teilchen eine Teilchengröße von weniger als 220 nm aufweisen, wie durch dynamische Lichtstreuung in einem mono- oder multi-dispergierten Zustand in einem wässrigen Medium gemessen.

4. Pharmazeutische Zusammensetzung nach einem der Ansprüche 1 bis 3 zur Verwendung als ein Medikament.

5. Pharmazeutische Zusammensetzung nach einem der Ansprüche 1 bis 3, zur Verwendung in der Krebsbehandlung durch magnetisches Drug Targeting unter Verwendung eines externen magnetischen Feldes.

6. Pharmazeutische Zusammensetzung zur Verwendung nach Anspruch 5, wobei die Zusammensetzung intraarteriell in einer therapeutisch effektiven Menge verabreicht wird, und/oder wobei die Positionierung und die Vaskularisation des Zielgewebes und die Positionierung zum Erreichen des arteriellen Systems durch angiographisches Imaging bestimmt werden.

7. Pharmazeutische Zusammensetzung zur Verwendung nach Anspruch 5 oder 6, wobei die Menge der pharmazeutischen Zusammensetzung, die zu verabreichen ist, mittels Online-Monitoring des Zielgewebes eingestellt wird und/oder wobei die Zusammensetzung durch einmalige Verabreichung oder durch wiederholte Verabreichung gefolgt von angiographischem und MRT-basiertem Monitoring verabreicht wird.

8. Pharmazeutische Zusammensetzung zur Verwendung nach einem der Ansprüche 5 bis 7, wobei die pharmazeutische Zusammensetzung zu dem Zielgewebe durch Anlegen eines externen magnetischen Feldgradienten mit einer hohen magnetischen Flussdichte im Bereich von 0,5 bis 1,5 T und einem Magnetfeldgradienten im Bereich von 10 bis 90 T/m geführt und angereichert wird.

9. Verfahren zum Herstellen einer pharmazeutischen Zusammensetzung nach einem der Ansprüche 1 bis 8, wobei das Verfahren zumindest die Schritte umfasst:
- Herstellen von magnetischen Eisenoxidnanoteilchen als die Teilchenkerne,
- Beschichten der Teilchenkerne mit Laurinsäure zumindest einmal, und
- Beladen von Mitoxantron auf die Oberfläche der Teilchenkerne und/oder in das Innere eines Aggregats der Teilchenkerne durch elektrostatische Wechselwirkung.

10. Verfahren zum Herstellen einer pharmazeutischen Zusammensetzung nach Anspruch 9, wobei die Nanoteilchen Eisenoxidteilchen sind, welche in einer wässrigen Lösung, die Eisen (II)- und Eisen (III)-Salze enthält, durch Zugeben einer Base ausgefällt werden.

11. Verfahren zum Herstellen einer pharmazeutischen Zusammensetzung nach Anspruch 9 oder 10, wobei das Verfahren ferner einen Schritt des Autoklavierens der Zusammensetzung umfasst.

12. Verfahren zum Herstellen einer pharmazeutischen Zusammensetzung nach einem der Ansprüche 9 bis 11, wobei das Verfahren ferner den Schritt des Trennens der Nanoteilchen umfasst, um Teilchen und/oder Teilchenagglomerate mit einer Größe von 220 nm oder mehr zu eliminieren.

## Revendications

1. Composition pharmaceutique adaptée pour un ciblage magnétique, la composition comprenant des nanoparticules magnétiques, un milieu de dispersion aqueux ayant une teneur en matières sèches allant de 8 à 25 mg/ml, les particules comprenant un noyau de particule magnétique et un revêtement comprenant un acide gras, où l'acide gras est de l'acide laurique, et où les noyaux de particules sont revêtus avec 25 à 45 parties en poids de l'acide gras par rapport à 100 parties en poids du noyau de particule, où le noyau de particule est un oxyde de fer, et où les particules magnétiques comprennent en outre la mitoxantrone, qui est collée directement sur la surface des noyaux de particules et/ou à l'intérieur d'un agrégat des noyaux de particules par interaction électrostatique.

2. Composition pharmaceutique selon la revendication 1, dans laquelle le noyau de particule est un couple Fe₂O₃/Fe₃O₄ mono-domaine et multi-domaine.

3. Composition pharmaceutique selon la revendication 1 ou 2, dans laquelle les particules magnétiques ont une taille de particule inférieure à 220 nm telle que mesurée par diffusion dynamique de la lumière dans un état mono-dispersé ou multi-dispersé en milieu aqueux.

4. Composition pharmaceutique selon l'une des revendications 1 à 3, destinée à être utilisée en tant que médicament.

5. Composition pharmaceutique selon l'une des revendications 1 à 3, destinée à être utilisée dans le traitement du cancer par vectorisation magnétique de médicament en utilisant un champ magnétique externe.

6. Composition pharmaceutique destinée à une utilisation selon la revendication 5, dans laquelle la composition est administrée par voie intra-artérielle en quantité thérapeutiquement efficace, et/ou dans laquelle le positionnement et la vascularisation du tissu cible et le positionnement pour pouvoir accéder au système artériel sont déterminés par imagerie angiographique.

7. Composition pharmaceutique destinée à une utilisation selon la revendication 5 ou 6, dans laquelle la quantité de la composition pharmaceutique devant être administrée est réglée au moyen d'une surveillance en ligne du tissu cible et/ou dans laquelle la composition est administrée par administration unique ou par des administrations répétées suite à une surveillance angiographique et une surveillance basée sur le MRT.

8. Composition pharmaceutique destinée à une utilisation selon l'une des revendications 5 à 7, dans laquelle la composition pharmaceutique est guidée vers le tissu cible et accumulée dans celui-ci par l'application d'un gradient de champ magnétique externe ayant une densité de flux magnétique élevée allant de 0,5 à 1,5 T et un gradient de champ magnétique allant de 10 à 90 T/m.

9. Procédé de fabrication d'une composition pharmaceutique selon l'une des revendications 1 à 8, le procédé comprenant au moins les étapes consistant à :
produire des nanoparticules magnétiques d'oxyde de fer comme étant lesdits noyaux de particules,
revêtir lesdits noyaux de particules avec de l'acide laurique au moins une fois, et
charger la mitoxantrone sur la surface des noyaux de particules et/ou à l'intérieur d'un agrégat des noyaux de particules par interaction électrostatique.

10. Procédé de fabrication d'une composition pharmaceutique selon la revendication 9, dans lequel les nanoparticules sont des particules d'oxyde de fer qui sont précipitées dans une solution aqueuse contenant des sels de fer(II) et de fer(III) par l'addition d'une base.

11. Procédé de fabrication d'une composition pharmaceutique selon la revendication 9 ou 10, dans lequel le procédé comprend en outre une étape d'autoclavage de la composition.

12. Procédé de fabrication d'une composition pharmaceutique selon l'une des revendications 9 à 11, dans lequel le procédé comprend en outre l'étape consistant à séparer les nanoparticules de manière à éliminer les particules et/ou les agglomérats de particules ayant une taille supérieure ou égale à 220 nm.
